# EUROPEAN PATENT APPLICATION

(11) **EP 4 249 589 A1**
(43) Date of publication of application: **27.09.2023**
(21) Application number: 23162832.2
(22) Date of filing: 20.03.2023
(51) Int. Cl.: C12N 5/0793, C12N 15/113

(54) **IN VITRO GENETIC DISEASE MODEL CELL AND METHOD FOR PRODUCING THE SAME**

(30) Priority: 22.03.2022 JP 2022046107
(71) Applicant: Ricoh Company, Ltd., Tokyo 143-8555 (JP)
(72) Inventor: IJICHI, Rie, Tokyo, 143-8555 (JP)
(74) Representative: Marks & Clerk LLP

(57) **Abstract**

An object of the present invention is to develop and provide a method for simply producing an *in vitro* genetic disease model cell exhibiting a high pathological reproducibility of a genetic disease and having the same gene background as a control cell. Provided is a method for producing an *in vitro* genetic disease model cell, including an expression inhibition step of inhibiting a responsible gene of a genetic disease of interest from being expressed in a subject differentiated cell derived from a pluripotent stem cell or an adult stem cell.

## Description

### CROSS-REFERENCE TO RELATED APPLICATIONS

The present application claims priority under 35 U.S.C. § 119 to Japanese Patent Application No. 2022-046107, filed March 22, 2022. The contents of which are incorporated herein by reference in their entirety.

### BACKGROUND OF THE INVENTION

### Field of the Invention

The present invention relates to an *in vitro* genetic disease model cell produced using an iPS cell-derived cell, and to a method for producing the model cell.

### Description of the Related Art

In recent years, a human genetic disease model including a human iPS cell-derived cell has been attracting attention as a material for drug development research. For example, in cases where a mutation in a disease-responsible gene is identified, an iPS cell is established from a patient-derived cell having the gene mutation, and induced to differentiate into a constituent cell of a tissue regarded as the disease subject. The differentiated cell, if found to have a disease-specific phenotype, can be utilized as a model cell of the genetic disease. An iPS cell established from a cell of a healthy person, which is used as a control, is different in the gene background, except for the disease-responsible gene, from the disease-derived iPS cell. This fact results in a problem in that the evaluation of a phenotype, for example, whether a disease-specific phenotype is due to a mutation in a responsible gene, is hardly determined. Depending on the subject disease, a cell derived from a patient can itself be very difficult to obtain, as is the case with a rare disease.

In cases where a disease cell is difficult to obtain, as above-mentioned, one method is to introduce a mutated gene of a disease-responsible gene into an iPS cell established from a cell of a healthy person, or to knock down or genome-edit a disease-responsible gene to acquire a genetic disease model iPS cell. Inducing the resulting genetic disease model iPS cell to differentiate into a tissue cell used as a disease subject makes it possible to produce a genetic disease model of interest. Since this method is relatively easy to obtain the original established iPS cell, the gene background can be equalized to evaluate a disease-specific phenotype more strictly. Inducing a genetic disease model iPS cell to differentiate into a tissue cell as a disease subject has technical difficulty, and also poses a problem in that the differentiation into a cell of interest takes a large amount of labor and time.

Rett syndrome is a genetic disease that is developed by a female child in early infancy, and age-dependently causes a symptom such as dystonia, postural movement abnormality, emotional disorder, mental retardation or epilepsy. For this syndrome without any radical treatment, the development of a new drug is strongly desired.

The responsible gene of Rett syndrome is a MECP2 gene mutation, and 80 to 90% of the patients have this mutated gene. There are many things unknown about the function of the MECP2 protein encoded by the MECP2 gene. To elucidate the function in detail, an *in vitro* Rett syndrome model using neurocyte has been produced hitherto. In addition, the MECP2 gene is known for being expressed in not only a neurocyte but also a glia cell such as an astrocyte. Although a glia cell is considered to be involved in the Rett syndrome development mechanism, a conventional *in vitro* Rett syndrome model is a single culture model of a neurocyte, posing a problem in that the function of an astrocyte remains to be verifiable.

Non-patent Document 1 discloses as follows: to analyze the involvement of an astrocyte in Rett syndrome, both iPS cells derived from a Rett syndrome patient having a verified mutation in the MECP2 gene and iPS cells derived from a healthy person were induced to differentiate into neurocytes and astrocytes; and these neurocytes and astrocytes of each of the MECP2 wild type and mutant were made into four combinations, which were each cocultured, with the result that the degree of the Rett syndrome-like phenotype was different among the combinations. As an effect obtained by adding a drug, an unexpected result was obtained during a coculture of the astrocyte differentiated from the iPSC derived from Rett syndrome and the neurocyte differentiated from the iPSC derived from a healthy person. In general, analyzing the function of the MECP2 gene in a neurocyte and an astrocyte and the Rett syndrome development mechanism encompasses scrutinizing a phenomenon due to the responsible gene. There is a problem in that a system including a patient-derived iPSC and a healthy person-derived iPSC is not free from the influence of the gene background. Establishing the iPS cell strain with the MECP2 gene knocked down or genome-edited in the strain is followed by inducing the cell strain to differentiate into a neurocyte, posing a problem of taking a large amount of labor and time.

### SUMMARY OF THE INVENTION

An object of the present invention is to develop a method for simply and promptly producing an *in vitro* genetic disease model cell exhibiting a high pathological reproducibility of a genetic disease and having the same gene background as a control cell, and to provide the model cell.

Another object of the present invention is to search for and select a candidate therapeutic drug for the disease using the *in vitro* genetic disease model cell.

To achieve the above-mentioned objects, the present inventors have made studies vigorously. As a result, the present inventors have discovered that, when a responsible gene of a genetic disease is inhibited from being expressed in a differentiated cell derived from a pluripotent stem cell or an adult stem cell, which is already differentiated to a genetic disease subject cell of interest, the differentiated cell exhibits the same phenotype as the cell derived from the patient with the disease. This differentiated cell, which is derived from the pluripotent stem cell or the adult stem cell and in which the responsible gene is inhibited from being expressed, can be produced simply as the genetic disease model cell without inducing the pluripotent stem cell or the adult stem cell to differentiate into the genetic disease subject cell, and also makes it possible to equalize the genetic background by using, as a control cell, the pluripotent stem cell or the adult stem cell used in the production. The present invention is based on the above-mentioned discovery, and is to provide the following.
(1) A method for producing an *in vitro* genetic disease model cell, including an expression inhibition step of inhibiting a responsible gene of a genetic disease of interest from being expressed in a subject differentiated cell derived from a pluripotent stem cell or an adult stem cell.
(2) A method for producing an *in vitro* Rett syndrome model cell, including an expression inhibition step of inhibiting a MECP2 gene from being expressed in at least one of a neurocyte and a glia cell that is derived from an iPS cell.
(3) A genetic disease model cell produced or obtainable using the method for producing an *in vitro* genetic disease model cell according to (1).
(4) A Rett syndrome model cell produced or obtainable using the method for producing an *in vitro* Rett syndrome model cell according to (2).
(5) A method for searching for a genetic disease therapeutic drug, including: an administration step of administering a candidate drug to the genetic disease model cell according to (3) or the Rett syndrome model cell according to (4); and a selection step of verifying a disease phenotype in the above-mentioned cell after the administration step, and selecting the candidate drug as a candidate therapeutic drug on the basis of the degree of improvement.

A method for producing an *in vitro* genetic disease model cell according to the present invention makes it possible to provide a method for simply producing an *in vitro* genetic disease model cell exhibiting a high pathological reproducibility of a genetic disease and having the same gene background as a control cell.

The present invention makes it possible to search for and select a candidate therapeutic drug for the disease using the *in vitro* genetic disease model cell.

### BRIEF DESCRIPTION OF THE DRAWINGS

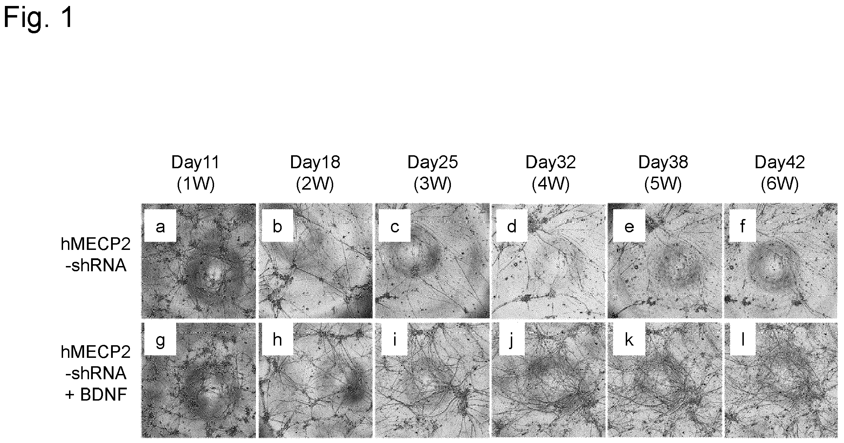

FIG. 1 is the micrographs illustrating a knockdown effect on hMECP2 by hMECP2-shRNA under a coculture of an iPS cell-derived differentiated neurocyte and a human primary astrocyte, "a" to "f" are the micrographs illustrating a chronological morphological change in a neurocyte infected with an hMECP2-shRNA lentivirus, and "g" to "l" are micrographs illustrating a chronological morphological change in a neurocyte infected with an hMECP2-shRNA lentivirus, in which a culture medium was supplemented with BDNF that is a therapeutic drug for Rett syndrome;

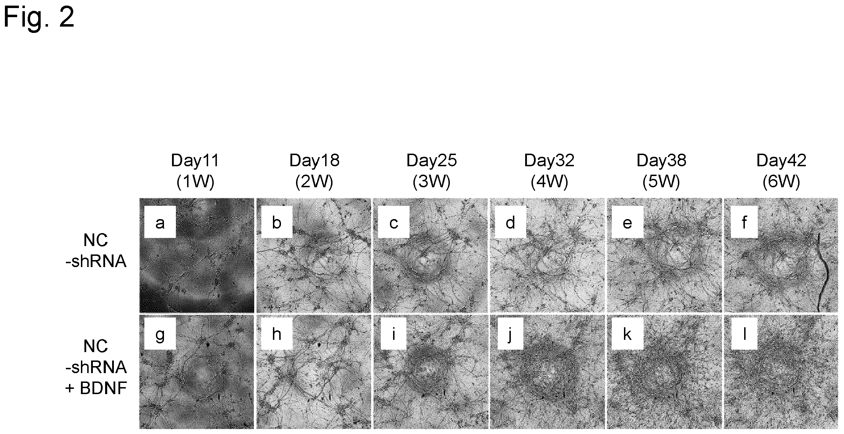

FIG. 2 is the micrographs illustrating an effect by NC-shRNA as a control under a coculture of an iPS cell-derived differentiated neurocyte and a human primary astrocyte, "a" to "f" are the micrographs illustrating a chronological morphological change in a neurocyte infected with an NC-shRNA lentivirus, and FIGs. "g" to "l" are the micrographs illustrating a chronological morphological change in a neurocyte infected with an NC-shRNA lentivirus, in which a culture medium was supplemented with BDNF that is a therapeutic drug for Rett syndrome;

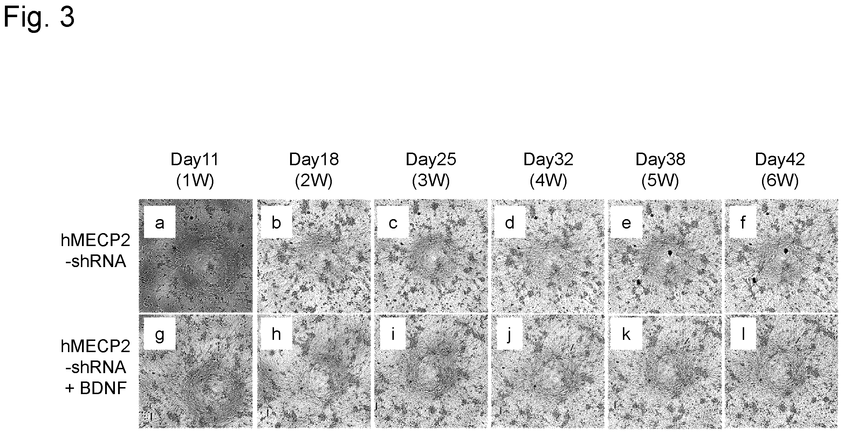

FIG. 3 is the micrographs illustrating a knockdown effect on hMECP2 by hMECP2-shRNA under a single culture of an iPS cell-derived differentiated neurocyte alone, "a" to "f" are the micrographs illustrating a chronological morphological change in a neurocyte alone infected with an hMECP2-shRNA lentivirus, and "g" to "l" are the micrographs illustrating a chronological morphological change in a neurocyte alone infected with an hMECP2-shRNA lentivirus, in which a culture medium was supplemented with BDNF that is a therapeutic drug for Rett syndrome;

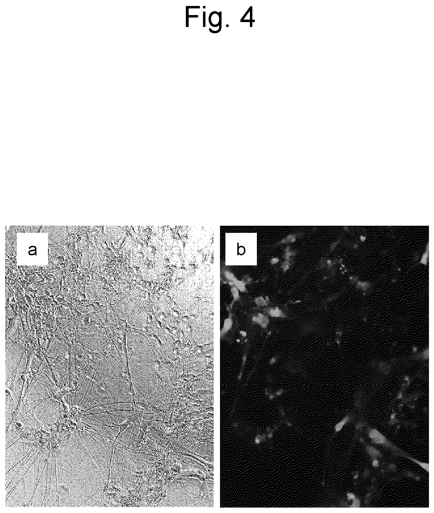

FIG. 4 illustrates an iPS cell-derived neurocyte in which hMECP2 was knocked down by decreasing the amount of infection with a lentivirus vector, "a" is a phase-contrast micrograph of the neurocyte, "b" is a fluorescence micrograph of the same field of view as "a", and a neurocyte infected with an hMECP2-shRNA lentivirus presents the fluorescence of an mCherry that is a marker for a lentivirus; and

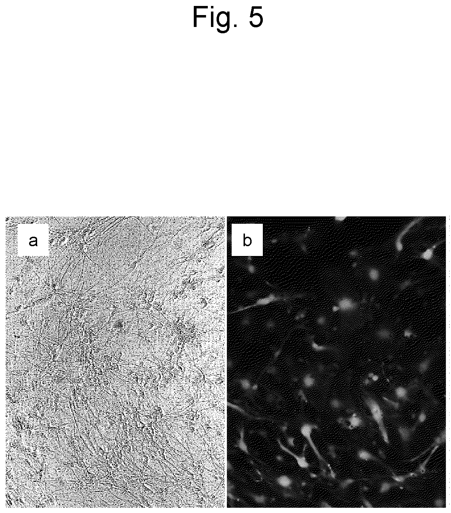

FIG. 5 illustrates an iPS cell-derived neurocyte in which hMECP2 was knocked down by increasing the amount of infection with a lentivirus vector, "a" is a phase-contrast micrograph of the neurocyte, "b" is a fluorescence micrograph of the same field of view as "a", and a neurocyte infected with an hMECP2-shRNA lentivirus presents the fluorescence of an mCherry that is a marker for a lentivirus.

### DESCRIPTION OF THE EMBODIMENTS

### 1. Method for Producing In Vitro Genetic Disease Model Cell

### 1-1. Overview

A first aspect of the present invention is a method for producing an *in vitro* genetic disease model cell (herein often referred to simply as a "production method" for short). The production method according to the present invention includes producing an *in vitro* genetic disease model cell by virtue of inhibiting a responsible gene of a genetic disease of interest from being expressed in a differentiated cell as a subject cell differentiated from a pluripotent stem cell or an adult stem cell. The production method according to the present invention makes it possible to produce and provide, in a simple manner, in a short time, and at a relatively low cost, an *in vitro* genetic disease model cell that exhibits the same phenotype as the genetic disease cell, and has the gene background equalized with control cell.

### 1-2. Definition of Terms

The terms used herein are defined below.

As used herein, an *"in vitro* genetic disease model cell" (herein often referred to simply as a "disease model cell" for short) refers to a cell that is cultured *in vitro* and exhibits the same phenotype as a specific genetic disease cell. As the cell reflects the same nature and character as a specific genetic disease, the cell can be a model cell of the specific genetic disease cell.

A "stem cell" is a cell that has a self-renewal ability which enables the cell to produce the same cell as the cell itself by division, that has a pluripotent differentiation potential which enables the cell to differentiate into a different cell lineage, and that has an unlimited proliferating ability. Examples of known stem cells include adult stem cells (also referred to as tissue stem cells or somatic stem cells) and pluripotent stem cells.

A "pluripotent stem cell" refers to a cell that has pluripotency (multipotency) by which the cell can differentiate into any kind of cell included in an organism, and that can unlimitedly proliferate in an *in vitro* culture under suitable conditions while maintaining the multipotency. Examples of such stem cells include iPS cells, ES cells, EG cells, and GS cells (germline stem cells). An "iPS cell" is a pluripotent stem cell that can be obtained by reprogramming, i.e., allowing a small number of genes encoding initialization factors to be introduced into a differentiated somatic cell so that the somatic cell can be brought into an undifferentiated state. An "ES cell" (embryonic stem cell) is a pluripotent stem cell produced from an early embryo. An "EG cell" (embryonic germ cell) is a pluripotent stem cell produced from a primordial germ cell of an embryo. A "GS cell" (induced pluripotent stem cell) is a pluripotent stem cell produced from a cell testis (Conrad, S., Nature, volume 456, 2008, pp. 344-349).

An "adult stem cell" is a stem cell that exists in each tissue of an adult, not terminally differentiated yet, and has some degree of pluripotency. An adult stem cell is also referred to as a somatic stem cell or a tissue stem cell. Specific examples of adult stem cells include mesenchymal stem cells, neural stem cells, intestinal epithelium stem cells, hematopoietic stem cells, hair follicle stem cells, and pigment stem cells. A "mesenchymal stem cell (MSC)" is a cell having differentiation potency into a cell belonging to a mesenchymal cell mainly such as osteoblast, adipocyte, myocyte, or chondrocyte. A "neural stem cell" is a cell having differentiation potency mainly into a neurocyte and a glia cell. An "intestinal epithelium stem cell" is a cell having differentiation potency into an epithelial cell included in the inner wall of a gastrointestinal tract mainly such as the small intestine or the large intestine. A "hematopoietic stem cell" is a cell having differentiation potency into a blood cell mainly such as an erythrocyte, a leucocyte, or a platelet. A "hair follicle stem cell" is a cell having differentiation potency into a follicular epithelial cell such as a hair stem cell or a hair root sheath cell, and besides, a sebaceous cell or a basal cell. Then, a "pigment stem cell" is a cell having differentiation potency mainly into a pigment cell.

Herein, "a pluripotent stem cell or an adult stem cell" is often referred to as "a pluripotent stem cell or the like".

Herein, a "differentiated cell" is a cell that has been induced to differentiate from a stem cell such as a pluripotent stem cell or an adult stem cell, and has undergone fate determination so as to become a specific cell.

Herein, a "subject cell" refers to a cell for producing a genetic disease model cell in the present invention, and is a cell included in a tissue or an organ which develops the symptom of a genetic disease as a phenotype. The kind of a subject cell depends on the genetic disease. For example, if the genetic disease of interest is a nervous disease, the subject cell is at least one of a neurocyte and a glia cell.

Herein, a "subject differentiated cell" refers to a differentiated cell as a subject cell differentiated from a pluripotent stem cell or the like.

A "genetic disease" (also referred to as a hereditary disease) is a generic term for diseases that are developed owing to the mutation of a chromosome or a gene. Examples of known genetic diseases include: familial genetic diseases having a character inheritable to next generations; and sporadic genetic diseases dispersively developed and not inheritable. Genetic diseases herein may be either familial genetic diseases or sporadic genetic diseases.

A "central nervous system disease" is a generic term for diseases induced by a structural lesion or a functional lesion in a nervous system such as the brain or the spinal cord. A central nervous system disease leads to developing various symptoms such as movement disorder, paralysis, epilepsy, neurodevelopmental disorder, muscle weakness, consciousness disorder, higher brain dysfunction, and psychiatric disorder. Specific examples of the neurodevelopmental disorder include Rett syndrome caused by a mutation of the MECP2 gene. In addition, specific examples of the psychiatric disorder include schizophrenia, depression, or bipolar disorder. Herein, neurodegenerative diseases are encompassed in central nervous system diseases. A neurodegenerative disease is a generic term for progressive nervous diseases developed by gradual diminishment and vanishment of a specific neurocyte or a glia cell in the central nervous system. A neurodegenerative disease can be a disease developed by the formation and accumulation of an abnormally proteinaceous inclusion body in a neurocyte or a glia cell, and besides, often be a disease the cause of which is not clear. Any of such diseases is intractable, and has no effective therapy. Many of the central nervous system diseases are hereditary diseases. A neurodegenerative disease as a subject herein is also a nervous disease belonging to hereditary diseases. Examples of neurodegenerative diseases include: Parkinson's disease caused by a mutation of the parkin gene; Huntington disease (HD) caused by a mutation of the huntingtin gene; Alzheimer disease (AD) caused by a mutation of the PSEN1 gene, the PSEN2 gene, or the APP gene; and frontotemporal lobar degeneration (Pick's disease, FTLD) caused by a mutation of the TDP-43 gene.

A "neurocyte" refers to a cell that is included in a nerve tissue and is specialized in information transmission. The basic structure of the cell has one axon and a plurality of dendrites. Examples of known neurocytes include sensory nerve cells, internal nerve cells, motor nerve cells and the like. A neurocyte herein may be any of the neurocytes.

A "glia cell" refers to a cell encompassed in a group of cells that are present in the central nervous system and the spinal cord, and involved in maintaining the neurocytes. Examples of known glia cells include an astrocyte, microglia, Schwann cell (sheath cell), oligodendrocyte, satellite cell and the like. A glia cell herein may be any of the glia cells.

A "responsible gene" as used herein refers to a gene that is directly responsible or indirectly responsible for any of the above-mentioned genetic diseases or central nervous system diseases. The gene is preferably directly responsible. For example, the responsible gene of Rett syndrome is the MECP2 gene. In principle, a genetic disease or a central nervous system disease herein is developed by the inhibition of the expression of the responsible gene or the inhibition of the function of the protein encoded by the gene.

A "gene expression vector" as used herein refers to an expression unit that contains a gene or a functional nucleic acid in an expressible state, and can control the expression of the gene or the like. Gene expression vectors that can be utilized are various expression vectors renewable and expressible in a host cell into which the vector is introduced, i.e., a pluripotent stem cell or the like or a subject differentiated cell. Examples of such vectors include virus vectors. Examples of virus vectors include various vectors derived from retroviruses, lentiviruses, adenoviruses, adeno-associated viruses and the like.

Herein, "inhibiting a gene from being expressed" or the "inhibiting (inhibition of) the expression of a gene" is also referred to as a gene knockdown, and refers to inhibiting the expression and function of the protein encoded by the gene. Specific examples of such inhibition include inhibiting the function of a transcription product (mRNA) or a translation product (protein) of a target gene during transcription, after transcription, during translation, or after translation in the expression of a target gene. Such inhibition is distinguished from gene knock-out, which disrupts a gene and completely obliterates the function of the protein to be encoded by the gene.

### 1-3. Production Method

A method for producing an *in vitro* genetic disease model cell according to the present invention includes a differentiation induction step and an expression inhibition step. Below, each step will be described specifically.

### 1-3-1. Differentiation Induction Step

The "differentiation induction step" is a step of inducing a pluripotent stem cell or the like to differentiate into a subject cell. The present step is an optional step in the production method according to the present invention, and can be performed, if desired. The present step makes it possible to obtain a subject differentiated cell, i.e., a subject cell differentiated from a pluripotent stem cell or the like.

In principle, the present step is performed before the below-mentioned expression inhibition step. A subject differentiated cell obtained in the differentiation induction step is used in the expression inhibition step. The present step can be performed simultaneously with the expression inhibition step. In this case, a pluripotent stem cell or the like is induced to differentiate into a subject cell by direct differentiation. In addition, the responsible gene of a genetic disease of interest is inhibited from being expressed.

The derivation organism of a pluripotent stem cell or the like to be used in the present step is not limited to any species. The species can be, for example, a mammal, a bird or the like. The mammal may be any one of primates (including humans and monkeys), rodents (including mice, rats, hamsters, and guinea pigs), bovines, horses, sheep, goats, dogs, cats, and the like and is preferably a human. In addition, the derivation individual of a pluripotent stem cell or the like is not limited to any gender, age, or health state. The health state may be a healthy state, or may be a state of contraction of a specific disease.

In addition, a method for obtaining a pluripotent stem cell or the like to be used in the present step is not limited to any such method. A commercially available cell or a transferred cell may be used, or a newly produced cell preferably of a clinical grade may be used. Without limitation, when used in each aspect of the present invention herein, a pluripotent stem cell is preferably an iPS cell or an ES cell, and an adult stem cell is preferably a mesenchymal stem cell or a neural stem cell.

In cases where a pluripotent stem cell or the like is newly produced, any known method for producing each stem cell can be used. For example, an iPS cell can be produced by combining the OCT3/4 gene, KLF4 gene, SOX2 gene, and c-Myc gene (Yu, J. et al., Science, volume 318, 2007, pp. 1917-1920), or combining the introduced OCT3/4 gene, SOX2 gene, LIN28 gene, and Nanog gene (Takahashi, K. et al., Cell, volume 131, 2007, pp. 861-872), and introducing the combination into a differentiated somatic cell.

In cases where a commercially available iPS cell is used, for example, a 253G1 strain, 201B6 strain, 201B7 strain, 409B2 strain, 454E2 strain, HiPS-RIKEN-1A strain, HiPS-RIKEN-2A strain, HiPS-RIKEN-12A strain, Nips-B2 strain, TkDN4-M strain, TkDA3-1 strain, TkDA3-2 strain, TkDA3-4 strain, TkDA3-5 strain, TkDA3-9 strain, TkDA3-20 strain, hiPSC 38-2 strain, MSC-iPSC1 strain, BJ-iPSC1 strain, RPChiPS771-2 strain, 1231A3 strain, 1210B2 strain, 1383D2 strain, 1383D6 strain or the like can be used.

In cases where a commercially available ES cell is used, for example, a KhES-1 strain, KhEs-2 strain, KhEs-3 strain, KhEs-4 strain, KhEs-5 strain, SEES1 strain, SEES2 strain, SEES3 strain, SEES-4 strain, SEEs-5 strain, SEEs-6 strain, SEEs-7 strain, HUES8 strain, CyT49 strain, H1 strain, H9 strain, HS-181 strain or the like can be used.

A method for inducing a pluripotent stem cell or the like to differentiate into a subject cell can be a method known in the art. Such a method is usually, for example, a method in which a differentiation-inducing factor is introduced into a pluripotent stem cell or the like, or a method in which a differentiation-inducing factor is added into a liquid culture medium for culturing a pluripotent stem cell or the like.

In the present invention, a differentiation inducer refers to a substance that promotes differentiation induction. Such a differentiation inducer is not limited to any kind. Examples of differentiation inducers include: a transcription gene encoding a transcription factor that activates the expression of a group of genes to be necessary for differentiation to a specific cell, or an mRNA of the transcription gene; an Activin/TGFβ signal inhibitor; BMP signal activator; BMP signal inhibitor; retinoic acid signal activator; hedgehog signal activator; hedgehog signal inhibitor; Notch signal inhibitor; WNT/β-catenin signal activator; JNK signal inhibitor; Src signal inhibitor; AMPK signal inhibitor; EGF signal activator; EGF signal inhibitor; HGF signal activator; VEGF signal activator and the like. Which transcriptional inducer is to be used is determined by into which subject cell a pluripotent stem cell or the like is induced to differentiate.

For example, in cases where an iPS cell is induced to differentiate into a skeletal muscle, a transcription gene cocktail of a MyoD gene, Pax7 gene/Pax3 gene, Mef2b gene, and Pitx1 gene can be included in an expression vector in an expressible state. The resulting vector can be introduced into an iPS cell. Alternatively, an mRNA cocktail of the genes may be introduced into an iPS cell. A method for introducing a transcription gene into a pluripotent stem cell or the like can be in accordance with a transformation method or a transfection method known in the art with respect to an expression vector to be used for the introduction. For example, in the case of a virus vector, a pluripotent stem cell or the like can be infected with a virus vector to be introduced into the vector. A transfection method using a virus vector will be described in detail in the below-mentioned expression inhibition step. In addition, the expression of a transcription gene is based on a promoter of an expression vector. In cases where the promoter is a constitutively active promoter, the present step is achieved by introducing an expression vector and then only culturing without any particular induction treatment. An expression induction promoter will undergo the culture in the present step and in addition, an induction treatment peculiar to the promoter. For example, in the case of a promoter containing TRE, tetracycline (Tet) as an expression inducer or doxycycline (Dox) as a derivative of tetracycline can be added to a culture medium to activate the promoter contained in the expression vector in the cell.

In cases where an iPS cell is induced to differentiate into a neurocyte, the method includes, for example, a method in which FGF-2 as a neurocyte differentiation-inducing factor is added into a culture medium for culturing a pluripotent stem cell or the like. FGF-2 (fibroblast growth factor-2) is a protein factor that stimulates cell growth of various kinds, and is involved in the differentiation, existence, and regeneration inductions of a nerve.

Herein, unless otherwise particularly specified, a culture of a pluripotent stem cell or the like or a subject differentiated cell can be performed under usual cell culture conditions, as is also the case with the below-mentioned expression inhibition step. The culture may be either an adhesion culture or a suspension culture. An "adhesion culture" is a culture in which cells are allowed to adhere to an external matrix such as a culture container, and grown in a single layer in principle. A "suspension culture" is a culture in which cells are grown in a culture medium without being allowed to adhere to an external matrix.

A culture medium to be used is not limited to any particular culture medium as long as such a culture medium contains a minimal or more component(s) essential for growth and maintenance of cells. Unless otherwise specified, the culture medium herein is an animal cell culture medium to be used to culture an animal-derived cell.

As used herein, an "animal cell culture medium" refers to a culture medium that is commonly used to culture an animal cell and known in the art. Either a basal culture medium or a special culture medium may be used. As used herein, a "basal culture medium" refers to a versatile culture medium to be used to culture various kinds of cells derived mainly from a mammal. Specific examples of basal culture media include Eagle MEM (Eagle Minimum Essential Medium), DMEM (Dulbecco's Modified Eagle Medium), Ham F10 (Ham's Nutrient Mixture F10) culture medium, Ham F12 (Ham's Nutrient Mixture F12) culture medium, M199 culture medium, high-performance improved 199 culture medium (Hight Performance Medium 199), and RPMI-1640 (Roswell Park Memorial Institute-1640) culture medium. A "special culture medium" refers to a culture medium so prepared by supplementing the basal culture medium with an additive as to be optimal for the culture of a specific cell, or a culture medium prepared to promote the induction of differentiation into a specific cell. Examples of special culture media include neurocyte culture media commercially available from life science manufacturers. Specific examples include a neurocyte culture medium from Sumitomo Bakelite Co., Ltd., i.e., a culture medium prepared by supplementing DMEM/F12 (at 5:5) with insulin and transferrin to prepare a basal culture medium, and then supplementing the basal culture medium with culture supernatant and serum albumin of a primary astroglia cell.

After a pluripotent stem cell or the like or a subject differentiated cell is seeded in a culture medium, a culture can be performed under culture conditions such as under 5% CO₂ at 37°C.

The culture period differs depending on the kind and derivation of a cell. In the case of a culture in the present step, the period is a time in which a pluripotent stem cell or the like completes differentiating into a subject cell. For example, the period can be from 5 weeks to 12 weeks, preferably from 6 weeks to 10 weeks, for the induction of differentiation from an iPS cell into a neurocyte. In addition, for the induction of differentiation from an iPS cell to an astrocyte, the period can be from 12 weeks to 30 weeks, preferably from 15 weeks to 20 weeks. For example, by direct differentiation, in cases where an iPS cell is induced to differentiate into a neurocyte, the period can be from 1 week to 2 weeks, and in cases where an iPS cell is induced to differentiate into an astrocyte, the period can be from 4 weeks to 8 weeks.

### 1-3-2. Expression Inhibition Step

The "expression inhibition step" is a step of inhibiting the responsible gene of a genetic disease of interest from being expressed in a subject differentiated cell derived from a pluripotent stem cell or the like. The present step is an essential step in the production method according to the present invention. In principle, a cell to be used in the present step is a subject differentiated cell that has completed differentiating into a subject cell. As above-mentioned, this principle does not apply in cases where the above-mentioned differentiation induction step is performed simultaneously with the present step.

The kind of a subject differentiated cell to be used can be determined in accordance with a genetic disease of interest. For example, if the genetic disease of interest is Rett syndrome, the subject differentiated cell can be at least one of a neurocyte and a glia cell.

In the present step, the responsible gene of a genetic disease of interest is inhibited from being expressed.

Herein, the MECP2 gene, which is the responsible gene of Rett syndrome, is used as an example of a target gene, and described below.

The MECP2 gene is the human MECP2 gene encoding the human MECP2 protein having the amino acid sequence of SEQ ID NO: 1, or a MECP2 gene mutation encoding an MECP2 protein having the same amino acid sequence as SEQ ID NO: 1 except that one or a plurality of amino acids are deleted, substituted, or added, or having an amino acid sequence having an amino acid identity of 90% or more, preferably 95% or more, 96% or more, 97% or more, 98% or more, or 99% or more, to the amino acid sequence of SEQ ID NO: 1. More specific examples of the human MECP2 gene include the human MECP2 gene having the base sequence of SEQ ID NO: 2 and encoding the human MECP2 protein having the amino acid sequence of SEQ ID NO: 1. Herein, "a plurality of' refers to, for example, from 2 to 20, from 2 to 15, from 2 to 10, from 2 to 7, from 2 to 5, from 2 to 4, or from 2 to 3. The "substitution (of an amino acid)" refers to a substitution in a group of conservative amino acids similar in properties such as electric charge, side chain, polarity, aromaticity and the like among 20 kinds of amino acids that are included in natural proteins. An "amino acid identity" refers to the ratio (%) of the number of the same amino acid residues in one amino acid sequence as in another amino acid sequence to the number of all the amino acid residues in the another amino acid sequence when the two amino acid sequences are arranged side by side (in alignment) with a gap introduced in one or both of the amino acid sequences, if desired, so that both the amino acids can have the highest degree of coincidence between the amino acids.

In the present step, a method to be used to inhibit the expression of a responsible gene can be a method known in the art without any particular limitation, and is preferably a gene knockdown method established technically and expected to afford a simple and high effect.

A "gene knockdown method" is a method for decreasing or depleting a target gene product (for example, an mRNA or protein) in a cell via a nucleic acid molecule (for example, at least one of the below-mentioned RNAi agent, antisense oligonucleotide, and nucleic acid aptamer). The target can be a gene product given after the transcription and before the translation of a target gene, that is, a gene transcription product such as an mRNA or the like, or the target can be a translation product given after the translation of the target gene, that is, a protein. In either of the cases, the effect of inhibiting the expression of a responsible gene is dependent on the amount of nucleic acid molecules to be introduced into cells. Adjusting the amount of nucleic acid molecules to be introduced makes it possible to adjust the efficiency at which the expression of the responsible gene is inhibited. For example, in cases where the nucleic acid molecules to be introduced into subject differentiated cells is a large amount, the expression of the responsible gene can be inhibited in most of the subject differentiated cells having the molecules introduced into the cells, but in the cases of the small amount of introduction, it is possible that, in the subject differentiated cells having the molecules introduced into the cells, the expression of the responsible gene is inhibited in a part of the cells, but not inhibited in another part. Genetic disease model cells in such a mixed state make it possible to reproduce a state approximate to an expression state of a responsible gene in a lesional tissue or the like of an actual patient with a genetic disease. Such cells are very preferable as genetic disease model cells.

Specific examples of gene knockdown methods include (1) an RNAi method, (2) an antisense oligonucleotide method, (3) a nucleic acid aptamer method and the like. Each method will be described below.

### (1) RNAi Method

The "RNAi method (RNA interference method)" is a method in which an RNAi agent is introduced into a cell as a host (host cell) so that the expression of a target gene is inhibited after transcription and before translation, or at a transcription stage, utilizing RNA interference (RNAi). RNA interference is sequence-specific gene silencing that inhibits the expression of a gene via the degradation or the like of a gene transcription product (mRNA) regarded as a target.

In cases where the expression of a responsible gene is inhibited using an RNAi method, an RNAi agent configured to induce gene silencing specific to a transcription product of the responsible gene is used herein. Specific examples of RNAi agents include an siRNA or an shRNA designed and artificially synthesized in accordance with a responsible gene. Each RNAi agent will be described below.

### (i) siRNA

An "siRNA" (small interference RNA) is a small-molecule duplex RNA including: an RNA sense strand (passenger strand) having a base sequence corresponding to a part of the sense strand of a target gene; and an RNA antisense strand (guide strand) that is the antisense strand of the RNA sense strand. Introducing an siRNA into a host cell makes it possible to induce RNA interference (Fire, A. et al., Nature, volume 391, 1998, pp. 806-811).

An siRNA can be designed with a known method on the basis of the base sequence of a target gene. For example, an siRNA can be designed on the basis of the method of Ui-Tei et al. (Nucleic Acids Res. RNAi, volume 32, 2004, pp. 936-948), the method of Reynolds et al. (Nat. Biotechnol., volume 22, 2004, pp. 326-330), or the method of Amarzguioui et al. (Biochem. Biophys. Res. Commun., volume 316, 2004, pp. 1050-1058).

In cases where the MECP2 gene is regarded as a target gene in the design of an siRNA, for example, as the base sequence of the passenger strand from the base sequence of SEQ ID NO: 2, a base sequence of consecutive 15 bases or more and 35 bases or less, preferably 15 bases or more and 30 bases or less, or 18 bases or more and 25 bases or less, is selected as a selected region. Attention is paid in such a manner that the base sequence of the region to be selected is completely identical to the base sequence of the target gene. The design is preferably such that the selected region does not encompass a known mutation of the target gene (for example, SNP or the like). The base sequence of the guide strand can be the base sequence complementary to the base sequence of the passenger strand selected as above-mentioned. When an siRNA is produced, the T (thymine) bases are converted to U (uracil) bases in the selected region of each of the passenger strand and the guide strand.

The selected region of the passenger strand is not limited to any particular region as long as the region has a sequence specific to a target gene. The region is preferably downstream from a region of at least 50 bases, more preferably from 70 bases to 100 bases, from the initiation codon. It is preferable to select a base sequence region having AA (adenine-adenine) at the 5' side in a candidate region of a passenger strand. The amount of GC (guanine-cytosine) contained in the selected region is preferably from 20 to 80%, more preferably from 30 to 70% or from 40 to 60%. The design of an siRNA is published on the website, and may be in accordance with such a publication. Inputting the base sequence of a target gene makes it possible to design an effective and suitable siRNA. Examples of typical siRNA design websites include siDirect (http://sidirect2.mai.jp/) and siDESIGN Center (https://horizondiscovery.com/en/ordering-and-calculation-tools/sidesign-center).

At one end or both ends of an siRNA, one or more base sequences not related to a base sequence of a target gene or a base sequence complementary to the base sequence of the target gene may be present. The number of such bases present at an end of an siRNA is preferably in the range of from 1 to 20 without any particular limitation. Specifically, for example, TT (thymine-thymine), UU (uracil-uracil), or the like is added to the 3' end side of each base strand.

### (ii) shRNA

An "shRNA" (short hairpin RNA) is a single-stranded RNA in which two RNA strands (a passenger strand and a guide strand) included in the siRNA are linked by a spacer sequence having a suitable base sequence. That is, an shRNA contains a region as a passenger strand and a region as a guide strand in one molecule, and has a structure such that the regions form a stem structure by virtue of mutual base pairing, and such that the spacer sequence has a loop structure to allow the molecule as a whole to have a stem-loop structure in hairpin form.

When an shRNA is introduced into a cell, the loop structure portion is cleaved, so that an siRNA is generated. The siRNA generated can inhibit the expression of a target gene by virtue of RNA interference described in the preceding section.

In the design of an shRNA, for example, the 3' end of the sense region and the 5' end of the antisense strand in the siRNA are linked by a spacer sequence. The spacer sequence can usually have 3 to 24 bases, preferably 4 to 15 bases. The spacer sequence is not limited to any particular sequence as long as the sequence enables the siRNA to undergo base pairing.

In an shRNA, a DNA encoding the shRNA can be inserted operably under the promoter control of an expression vector. Introducing such an shRNA expression vector into a target organism or a target cell allows an shRNA to be expressed by the activity of the promoter. After being expressed, the shRNA is processed into an siRNA via self-folding in a host cell or the activity of Dicer or the like. The effect of the siRNA is then exerted. In cases where the expression of a responsible gene is inhibited using an RNAi interference method in the present invention, an shRNA is particularly preferable.

A method for introducing an RNAi agent into a host cell is not limited to any particular method. A known introduction technology can be selected suitably in accordance with the kind and form of an RNAi agent to be introduced and the kind of a host cell. For example, as an introduction method using a virus vector containing an shRNA, a transfection method can be used for introduction into a host cell. In cases where a lentivirus vector or a retrovirus vector is used, an shRNA can be incorporated into the genome of a host cell. Thus, a pluripotent stem cell or the like or a subject differentiated cell, having an shRNA introduced into the cell, can express an shRNA stably for a long period of time, and continue to inhibit the expression of a responsible gene. Such a vector is convenient. Examples of methods using no virus vector include physicochemical methods such as a lipofection method, electroporation method, calcium phosphate method, DEAE-Dextran method, microinjection method and the like. These are all known technologies, and can be performed with reference to an existing protocol. For example, a gene transfer method described in Green & Sambrook, Molecular Cloning: A Laboratory Manual, fourth edition, 2012, Cold Spring Harbor Laboratory Press, Cold Spring Harbor, New York and the like can be referred to. A commercially available transfection reagent such as LIPOFECTAMINE^{™} 3000 (Thermo Fisher Scientific Inc.) may be used.

### (2) Antisense Oligonucleotide Method

An "antisense oligonucleotide" (herein often referred to as an "ASO") is a single-stranded nucleic acid molecule that has a base sequence complementary to all or part of the base sequence of an mRNA as a transcription product of a target gene, and hybridizes to a target mRNA to inhibit the translation of the mRNA.

A DNA ASO and an RNA ASO are known. The DNA ASO method is a nuclease-mediated type of gene inhibition method in which such an ASO hybridizes to an RNA molecule such as an mRNA as a transcription product of a target gene to form a hetero duplex structure, and then, the target RNA molecule is cleaved and degraded by the RNase H activity in the cell to inhibit the expression of the target gene. In the RNA ASO method, an ASO hybridizes to an RNA molecule such as an mRNA as a transcription product of a target gene to form a duplex RNA, then undergoes processing, and finally makes it possible to achieve the effect of inhibiting the expression of a gene by virtue of RNAi in the same manner as an siRNA. Without limitation, a DNA ASO method is usually often used because of the stability in a cell and the easiness of synthesis.

The DNA ASO includes mainly a naturally occurring DNA, and can partially contain at least one of the following: a nucleic acid analogue such as a naturally occurring RNA, LNA/BNA (Locked Nucleic Acid/Bridged Nucleic Acid), PMO (Phosphorodiamidate Morpholino Oligomers) or the like; and a modified nucleic acid such as 2'-OMe-RNA, 2'-F-RNA, 2'-MOE-RNA, phosphorothioate or the like. The DNA ASO encompasses an ASO having a special structure, such as: a gapmer as an RNA-degrading ASO having, at the 5' end and the 3' end, a wing region including a nucleic acid analogue or a modified nucleic acid; or a mixmer as a splicing control ASO including a nucleic acid analogue or a modified nucleic acid.

A hetero duplex oligonucleotide (HDO) method based on applying a DNA ASO method can also be used. A "hetero duplex oligonucleotide" (herein often referred to as a "HDO") is a duplex nucleic acid including the main strand (DNA strand) having an ASO function and a cRNA (complementally RNA) strand having the base sequence complementary to the main strand. A cRNA strand can also contain a nucleic acid analogue or a modified nucleic acid. All or part of at least the central portion of an HDO is a hetero nucleic acid including a DNA and an RNA. Thus, a cRNA strand as a complementary strand is cleaved and degraded by an RNase H in a cell. The main strand made alone can function as an ASO.

An RNA ASO has the same base sequence as an antisense strand that can hybridize to part of an mRNA or the like as a transcription product of a target gene, and includes a naturally occurring RNA, in principle. Because of this, the RNA ASO is herein often referred to as an "antisense RNA (asRNA)" to be distinguished from a DNA ASO.

In an asRNA, a DNA encoding the asRNA can be inserted operably under the promoter control of an expression vector. As with the above-mentioned shRNA expression vector, introducing such an "antisense RNA expression vector (asRNA expression vector)" into a target organism or a target cell allows an asRNA to be expressed by the activity of the promoter. For example, the asRNA after being expressed hybridizes to a transcription product of a target gene in a host cell. The effect of the asRNA is then exerted.

For example, in cases where the MECP2 gene is regarded as a target gene, regardless of a DNA and an RNA, specific examples of the design of the base sequence of the ASO include selecting a base sequence complementary to a base sequence of consecutive 10 bases or more and 30 bases or less, preferably 12 bases or more and 25 bases or less, or 13 bases or more and 20 bases or less, selected from the base sequence of SEQ ID NO: 2 for an mRNA strand as a selected region. In this case, for example, a region encompassing an initiation codon or a region capable of forming a single-stranded structure in a predictable secondary structure of an mRNA strand can be selected as a target region.

A method for introducing an ASO is in accordance with the above-mentioned method for introducing an siRNA. In addition, as above-mentioned, an asRNA can be introduced in a DNA state as an asRNA expression vector in the same manner as the shRNA expression vector so that an asRNA can be expressed in a host cell. A method for introducing an asRNA expression vector may also be in accordance with the method for introducing an shRNA expression vector.

### (3) Aptamer Method

An "aptamer method" is a method in which a nucleic acid aptamer is administered to a host cell, and the target binding activity of the aptamer inhibits a target protein from functioning, whereby the expression of a target gene is inhibited after translation. For example, if a genetic disease of interest is Rett syndrome, a nucleic acid aptamer herein is bound to a mutated MECP2 protein to inhibit the function of the protein, inhibiting the expression of the MECP2 gene after translation.

A "nucleic acid aptamer" is a ligand molecule that includes a nucleic acid and that is bound firmly and specifically to a target protein by a stereostructure formed on the basis of a secondary structure or even a tertiary structure of a single-stranded nucleic acid molecule via hydrogen bonding or the like, and inhibits the function of the target protein. A nucleic acid aptamer has the same action effect as an antibody, generally has a higher specificity and affinity to a target protein than an antibody, may have a smaller number of target amino acid residues required for bonding than an antibody, and then is better than an antibody in terms of the capability to distinguish among related molecules. A nucleic acid aptamer has an advantage in that the aptamer has a lower immunogenicity and toxicity than an antibody, additionally can be produced in a short period of approximately from 3 to 4 weeks, and besides, can be produced in large amounts by chemical synthesis.

As nucleic acid aptamers, an RNA aptamer including an RNA and a DNA aptamer including a DNA are known in general. A nucleic acid included in a nucleic acid aptamer herein is not limited to any particular nucleic acid. Examples of aptamers include DNA aptamers, RNA aptamers, and DNA/RNA hybrid aptamers. The constituent of an aptamer is usually a naturally occurring nucleic acid (DNA or RNA), but an aptamer may contain a non-naturally occurring artificial nucleic acid or a modified nucleic acid as a part of the aptamer. The base length of a nucleic acid aptamer according to the present invention is preferably in the range of from 10 bases to 100 bases without any limitation. The base length is more preferably in the range of from 15 bases to 80 bases. An aptamer is based on a known technology, and can be referred to, for example, Janasena, Clinical Chemistry volume 45, 1999, pp. 1628-1650 for detail.

A nucleic acid aptamer can be produced by a known technology. An RNA aptamer can be produced, for example, by sorting in a test tube using the SELEX (systematic evolution of ligands by exponential enrichment) method (Proc. Natl. Acad. Sci. U.S.A., volume 92, 1995, pp. 11509-11513).

A method for introducing a nucleic acid aptamer is in accordance with the above-mentioned method for introducing an siRNA. In cases where a nucleic acid aptamer includes a naturally occurring RNA, a DNA encoding the aptamer can be inserted operably under the promoter control of an expression vector. As with the above-mentioned shRNA expression vector, introducing such an "RNA aptamer expression vector" into a target organism or a target cell allows an RNA aptamer to be expressed stably by the activity of the promoter.

### 1-4. Effects

A method for producing an *in vitro* genetic disease model cell according to the present invention makes it possible to simply and promptly produce an *in vitro* genetic disease model cell exhibiting a high pathological reproducibility of a genetic disease and having the same gene background as a control cell.

A method for producing an *in vitro* genetic disease model cell according to the present invention makes it possible to obtain a group of cells as a mixture of the following: cells in which the expression of a responsible gene is decreased; and cells in which such expression is on a usual level. Such a group of cells is in a state close to a state where a responsible gene is expressed in the group of cells of an actual patient with a genetic disease, and can provide a model cell that has reproduced the pathology of a genetic disease to a higher degree than an *in vitro* genetic disease model cell according to a conventional method.

An *in vitro* genetic disease model cell produced by the production method according to the present invention is effective for a disease developed by dysfunction due to the deletion, underexpression, or mutation of a responsible gene.

### 2. Method for Searching for Therapeutic Drug for Genetic Disease

### 2-1. Overview

A second aspect of the present invention is a method for searching for a therapeutic drug for a genetic disease. A search method according to the present invention makes it possible to select a candidate drug that can improve a pathological disease phenotype by administering the candidate drug using a genetic disease model cell obtained by the production method according to the first aspect.

2-2. Method

A search method according to the present invention includes an administration step and a selection step as fundamental steps. Each step will be described below.

### 2-2-1. Administration Step

The "administration step" is a step of administering a candidate drug to a genetic disease model cell according to the first aspect. As a genetic disease model cell, a model cell that exhibits the pathology of a genetic disease for which a new drug is to be developed can be selected. For example, to develop a therapeutic drug for Rett syndrome without any radical treatment, a Rett syndrome model cell is used.

A candidate drug to be administered is not limited to any kind. Examples of the kind of such a drug include nucleic acids, peptides (encompassing proteins), and low-molecular-weight compounds. An administration method can be suitably determined in accordance with the kind of a candidate drug. For example, in the case of a nucleic acid or a peptide, a transfection method using a virus vector or the like according to the first aspect, or a physicochemical method such as a lipofection method, electroporation method, calcium phosphate method, DEAE-Dextran method, microinjection method or the like can be used. A low-molecular-weight compound can be added to a culture medium.

After a candidate drug is administered, the genetic disease model cell can be further cultured, if desired. A culture medium to be used is suitably determined in accordance with the kind of a genetic disease model cell. A culture medium has been described in detail in the first aspect section, and can be in accordance with the detail.

### 2-2-2. Selection Step

The "selection step" is a step in which a disease phenotype in the cell after the administration step is verified, and on the basis of the degree of improvement, a candidate drug is selected as a candidate therapeutic drug.

The disease phenotype can be any phenotype that a genetic disease model cell to be used has. For example, a Rett syndrome model cell exhibits a phenotype for a decrease in the number and length of neurites in a neurocyte. Whether this phenotype is rescued by administration of a candidate drug is verified in the present step. When this is verified, an iPS cell having the same genetic background as a subject differentiated cell used in the production of a genetic disease model cell, or a subject differentiated cell derived from the iPS cell may be used as a control, if desired. In cases where any improvement has been recognized, how much the improvement has been made is defined as a degree of improvement. For example, the recovery of 80% or more of a disease phenotype to a wild-type phenotype is determined to be a high degree of improvement, 60% or more and less than 80% to be a medium degree of improvement, and less than 60% to be a low degree of improvement. A candidate drug that has exhibited an effect corresponding to a medium or higher degree of improvement is selected as a candidate therapeutic drug. Then, in a nonclinical test, the candidate therapeutic drug selected can be tested for a medicinal effect and safety in another more detailed *in vitro* evaluation system or *in vivo* evaluation system.

### EXAMPLES

### <Example 1: Production of Rett Syndrome Model Cell>

### (Purpose)

To produce a Rett syndrome model cell using a method for producing an *in vitro* genetic disease model cell according to the present invention.

### (Method)

### (1) Production of Lentivirus for Expression of hMECP2-shRNA

Lenti-X 293T cells were seeded at 5.0 × 10⁶ cells/10 mL/dish in a 100 mm cell culture dish, and cultured under 5% CO₂ at 37°C in an incubator overnight. As the culture medium, DMEM (#11995065, Thermo Fisher Scientific Inc.) supplemented with 10% FBS and 1% penicillin-streptomycin was used.

As a lentivirus vector for transfection, an hMECP2-shRNA lentivirus vector plasmid was used. This plasmid vector contains, downstream of a U6 promoter, a base sequence of SEQ ID NO: 3 (gggtagggctctgacaaagcttcccgattaactgaaataaa) encoding an shRNA complementary to the 3' UTR of the hMECP2 gene. Additionally, as a control lentivirus vector, an NC-shRNA lentivirus vector plasmid was used. This plasmid vector contains, downstream of a U6 promoter, a non-mammalian shRNA having a base sequence of SEQ ID NO: 4 (gaaacaccggcaacaagatgaagagcaccaactcgagttgg).

On the day after the seeding, the lentivirus vector plasmid and a Lentiviral High Titer Packaging Mix were transfected into the Lenti-X 293T cell. Specifically, 1.5 mL of DMEM was supplemented with 7 µL of Lentiviral High Titer Packaging Mix and 11 µL of 0.5 µg/µL lentivirus vector, the resulting mixture was pipetted for complete mixing, and then supplemented with 45 µL of TransIT-293 Transfection Reagent. The resulting mixture was gently pipetted for mixing. Then, the resulting mixture was left to stand at room temperature for 20 minutes. All of the lentivirus liquid mixture was dropwise transfected into the Lenti-X 293T cell seeded the previous day. The Lenti-X 293T cells continued to be cultured under 5% CO₂ at 37°C in the incubator. At 24 hours after the transfection, the culture medium was replaced with 10 mL of a new culture medium. As the new culture medium, DMEM supplemented with 10% FBS and 1% penicillin-streptomycin was used.

At 48 hours after the transfection, a culture supernatant containing the lentivirus was collected. The culture supernatant collected was filtrated through a 0.45 µm filter. Then, the filtrate was supplemented with Lenti-X Concentrator in a 1/3 amount of the filtrate, and the resulting mixture was gently mixed, and incubated at 4°C for 6 hours. Then, the resulting mixture was centrifuged at 1500 g for 45 minutes to remove the supernatant. The pellets were suspended in 100 µL of DMEM. The resulting suspension was used as a virus liquid in a subsequent experiment.

### (2) Measurement of Virus Titer

HEK293T cells were seeded at 3 × 10³ cells/well in a 96-well plate. On the day after the seeding, the virus liquid prepared in (1) was diluted with DMEM 1-fold, 10-fold, 100-fold, 1000-fold, and 10000-fold. The liquids were each added at 10 µL/well. At 7 days after the infection, the HEK293T cells were observed under a fluorescence microscope. The ratio of the cells that had expressed mCherry, a marker gene of a lentivirus vector plasmid, was calculated.

As a result, the condition where 10 µL of the 10-fold diluted virus liquid was added to the 3 × 10³ cells in substantially 100% of which the expression of the mCherry was verified was denominated MOI1.

### (3) Culture of Neurocyte and Astrocyte

To a 384-plate (#142761, Thermo Fisher Scientific Inc.) for cell culture, 0.002% PLO/PBS was added at 30 µL/well. The wells were thus coated at 37°C for 1 hour. Then, the wells were washed with 100 µL/well distilled water three times, and dried. Then, 10 µg/mL Laminin was added at 30 µL/well to the wells, which were coated at 37°C for 1 hour.

Commercially available iPS-cell-derived neurocytes (#EX-SeV-CW50065, Elixirgen Scientific, Inc.) and commercially available human primary astrocytes (#N7805-100, Thermo Fisher Scientific Inc.) were thawed and used to prepare cell suspensions with culture media at 1 × 10⁴ cells/well and 2.5 × 10³ cells/well respectively. After the Laminine solution was removed, the suspension was seeded at 50 µL/well, and cocultured. For single culture, 1 × 10⁴ cells/well neurocytes were seeded at 50 µL/well. The culture continued under 5% CO₂ at 37°C in the incubator for 6 weeks. Half the amount of the culture medium was replaced once in 3 to 4 days. The culture medium used was a culture medium specified by Elixirgen Scientific, Inc. for the period of 1 week after the seeding. Then, the culture medium used was Neurobasal Plus supplemented with 2% B-27 Plus, 1% GlutaMAX, 200 µM ascorbic acid, 10% Neurocyte culture medium, and 1% penicillin-streptomycin.

### (4) Viral Infection and Rescue Experiment

At 4 days after the cell seeding, virus solutions for expression of the respective shRNAs were added under MOI 0.1 and MOI 1, and allowed to cause infection. MOI was prepared in accordance with only the number of neurocytes. At 7 hours after the infection, the whole amount of culture medium was replaced. To the culture medium in the wells to be rescued, BDNF, which is known for the therapeutic effect for Rett syndrome, was added at 50 ng/mL.

### (5) Observation of Cells

The form of the cells was observed under a phase-contrast microscope over 6 weeks, generally once a week (on Day 11, Day 18, Day 25, Day 32, Day 39, and Day 42).

### (Results)

FIGs. 1 to 3 illustrate a chronological morphological change in an iPS cell-derived differentiated neurocyte infected with a lentivirus. In addition, FIGs. 4 and 5 illustrate what the ratio of cells with hMECP2 knocked down in the cells was when the amount of infection with a lentivirus was regulated.

FIG. 1 illustrates a knockdown effect that hMECP2-shRNA had on hMECP2 when an iPS cell-derived differentiated neurocyte was infected with an hMECP2-shRNA lentivirus under a coculture of the neurocyte and a human primary astrocyte. The knockdown effect on hMECP2 according to the number of neurites and the length of the neurite in the neurocyte was evaluated. As a result, the neurocytes in "a" to "f" made it possible to verify a chronological decrease in the number of neurites and decrease in the length of the neurite that were caused by the knockdown of hMECP2. This knockdown effect in the neurocyte was improved by supplementing a culture medium with BDNF that is a therapeutic drug for Rett syndrome, as illustrated in "g" to "l". In FIG. 2, which illustrates the neurocytes infected with an NC-shRNA lentivirus as a control, neither a decrease in the number of neurites or a decrease in the length of the neurite was recognized. The above results have revealed that knocking down hMECP2 prepared using a method for producing an *in vitro* genetic disease model cell according to the present invention makes it possible not only to reproduce such pathology of a neurocyte as observed in Rett syndrome, but also to obtain a rescue effect through adding a therapeutic drug to the model cell. This suggests that an *in vitro* genetic disease model cell according to the present invention can be utilized to search for a therapeutic drug for a genetic disease.

FIG. 3 illustrates a chronological morphological change in an iPS cell-derived differentiated neurocyte infected with a hMECP2-shRNA lentivirus under a single culture. Interestingly, the single culture of the neurocyte exhibited neither a decrease in the number of neurites nor a change in the length of the neurite, and did not demonstrate a knockdown effect on hMECP2. This suggests that a decrease in the number of neurites in a neurocyte, a decrease in the length of the neurite and the like in Rett syndrome are developed by a coordinate influence of an abnormality of MECP2 in the neurocyte and an abnormality of MECP2 in the astrocyte. This result agrees with a report (Non-patent Document) made on Rett syndrome previously.

FIG. 4 and FIG. 5 are micrographs illustrating the relationship between the amount of infection with a hMECP2-shRNA lentivirus and the ratio of iPS cell-derived differentiated neurocytes with MECP2 knocked down in the neurocytes under a coculture of the neurocytes and primary astrocytes. FIG. 4 illustrating the infection with a small amount of lentivirus verifies that a knockdown effect on MECP2 was obtained in a part of the neurocytes, and that, in terms of the expression of MECP2, normal cells and abnormal cells were in a mixed state. In the case of FIG. 5 illustrating the infection with a large amount of lentivirus, a knockdown effect on MECP2 was achieved in substantially all the neurocytes by virtue of the high infection efficiency. These results demonstrate that the MECP2 knockdown efficiency dependent on hMECP2-shRNA can be controlled by the amount of viral infection. In the nerve tissues of a patient with Rett syndrome, normal cells and abnormal cells are mixed in terms of the expression of MECP2. Thus, an *in vitro* genetic disease model cell according to the present invention makes it possible to more accurately reproduce the pathology of an actual patient with Rett syndrome.

### Related Art Document

### [Non-patent Document]

[Non-patent Document 1] Williams, E.C., et al., Human Molecular Genetics, volume 23, issue 11, Jun 1, 2014, pp. 2968-2980

## Claims

1. A method for producing an *in vitro* genetic disease model cell, comprising
an expression inhibition step of inhibiting a responsible gene of a genetic disease of interest from being expressed in a subject differentiated cell derived from a pluripotent stem cell or an adult stem cell.

2. The method according to claim 1, further comprising
a differentiation induction step of inducing a pluripotent stem cell or an adult stem cell to differentiate into the subject differentiated cell.

3. The method according to claim 2, wherein the differentiation induction step is performed simultaneously with the expression inhibition step.

4. The method according to any one of claims 1 to 3, wherein the expression of the responsible gene is inhibited using a gene knockdown method.

5. The method according to claim 4, wherein the gene knockdown method is one or more methods selected from the group consisting of an RNAi method, an antisense oligonucleotide method, and a nucleic acid aptamer method.

6. The method according to any one of claims 1 to 5,
wherein the genetic disease of interest is a central nervous system disease, and
wherein the subject differentiated cell is a neurocyte.

7. The method according to claim 6, wherein the subject differentiated cell further comprises a glia cell.

8. The method according to claim 6 or 7, wherein the central nervous system disease is Rett syndrome, and wherein the responsible gene is an MECP2 gene.

9. The method according to claim 8, wherein the MECP2 gene has a base sequence encoding an MECP2 protein having an amino acid sequence of the following (a) to (c):
(a) an amino acid sequence of SEQ ID NO: 1;
(b) an same amino acid sequence as SEQ ID NO: 1 except that one or a plurality of amino acids are deleted, substituted, or added; or
(c) an amino acid sequence having an amino acid identity of 90% or more to the amino acid sequence of SEQ ID NO: 1.

10. The method according to claim 9, wherein the MECP2 gene has the base sequence of SEQ ID NO: 2.

11. The method according to any one of claims 1 to 10, wherein the pluripotent stem cell is an iPS cell or an ES cell.

12. A genetic disease model cell obtainable using the method for producing an *in vitro* genetic disease model cell according to any one of claims 1 to 7.

13. The genetic disease model cell according to claim 12, wherein the model cell is a Rett syndrome model cell obtainable using the method according to any one of claims 8 to 11.

14. A group of cells comprising, as part of the group of cells, the genetic disease model cell according to claim 12 or the Rett syndrome model cell according to claim 13.

15. A method for searching for a therapeutic drug for a genetic disease, comprising:
an administration step of administering a candidate drug to the genetic disease model cell according to claim 12 or the Rett syndrome model cell according to claim 13; and
a selection step of verifying a disease phenotype in the cell after the administration step, and selecting the candidate drug as a candidate therapeutic drug on the basis of the degree of improvement.
